# EUROPÄISCHE PATENTSCHRIFT

(11) **EP 1 720 467 B1**
(45) Veröffentlichungstag und Bekanntmachung des Hinweises auf die Patenterteilung: **14.09.2011**
(21) Anmeldenummer: 05716724.9
(22) Anmeldetag: 17.02.2005
(51) Int. Cl.: A61B 17/22

(54) **ANLAGE ZUR BILDGESTÜTZTEN STOSSWELLENBEHANDLUNG**
INSTALLATION USED FOR IMAGE-ASSISTED SHOCKWAVE THERAPY
EQUIPEMENT DE TRAITEMENT PAR ONDES DE CHOC A ASSISTANCE VISUELLE

(30) Priorität: 01.03.2004 DE 102004010005
(43) Veröffentlichungstag der Anmeldung: 15.11.2006
(73) Patentinhaber: SIEMENS AKTIENGESELLSCHAFT, 80333 München (DE)
(72) Erfinder: LANSKI, Markus, 90782 Fürth (DE); KALTSCHMIDT, Rainer, 90542 Eckental (DE); POLSTER, Walter, 91054 Erlangen (DE); FADLER, Franz, 91077 Hetzles (DE)
(86) Internationale Anmeldenummer: PCT/EP2005/050704
(87) Internationale Veröffentlichungsnummer: WO 2005/082261

(56) Entgegenhaltungen:
- DE-U1- 29 824 080
- US-A- 5 044 354
- US-A- 5 065 741
- US-A1- 2003 078 523

## Beschreibung

Die Erfindung betrifft eine Anlage zur bildgestützten Stoßwellenbehandlung. Die Hauptbestandteile einer solchen Anlage sind ein Therapiesystem und ein Röntgensystem. Das Therapiesystem umfasst einen Stoßwellenkopf, der auf einen Fokuspunkt gerichtete Ultraschallwellen erzeugt. Als Therapiezweck kommt vor allem die Zertrümmerung von Nieren- und Harnleitersteinen in Frage. Denkbar sind aber auch Anwendungen zur Behandlung der Induratio penis plastica oder im Bereich der Schmerztherapie und der Gastroenterologie. Das Röntgensystem dient zur Ortung des Steins im Behandlungsgebiet eines Patienten und zur therapiebegleitenden Beobachtung des Behandlungserfolges. Es umfasst eine Röntgenstrahlquelle und einen Röntgenempfänger bzw. Bildverstärker. Die beiden Geräte sind an den Schenkelenden eines um sein Isozentrum orbital verfahrbaren C-förmigen Bogens, im weiteren Röntgen-C-Bogen genannt, fixiert. Der Röntgen-C-Bogen umgreift im Anwendungsfall teilweise einen Patiententisch bzw. ist in Richtung einer rechtwinklig zu seiner Orbitalebene verlaufenden Achse von diesem durchsetzt.

Bei der Behandlung eines Patienten mit einer Anlage der oben geschilderten Art muss der Fokuspunkt des Stoßwellenkopfes auf das Isozentrum des Röntgen-C-Bogens ausgerichtet sein bzw. mit diesem zusammenfallen, damit bei einer zur 3D-Ortung erforderlichen orbitalen oder angularen Verfahrung des Röntgensystems dessen Strahlachse stets durch den Fokuspunkt bzw. durch einen diesen beinhaltenden Volumenbereich verläuft. Im Anwendungsfall muss dementsprechend das zu behandelnde Therapieobjekt ebenfalls in dem genannten Bereich angeordnet, d.h. der Patient in geeigneter Lage auf dem Patiententisch positioniert werden. Bei Anlagen mit feststehendem Stoßwellenkopf kann diese Forderung nur durch eine für den Patienten unangenehme Lage, beispielsweise durch eine insbesondere bei adipösen Patienten kritische Bauchlage erfüllt werden.

Bei einer aus DE 298 24 080 U1 bekannten Anlage ist in der Orbitalebene eines ausschließlich angular verschwenkbaren Röntgen-C-Bogens eine als C-Bogen ausgebildete Tragvorrichtung für einen Stoßwellenkopf angeordnet. Der C-Bogen umfasst ein erstes, am Röntgen-C-Bogen fixiertes Bogensegment und ein an diesem Segment verschiebbar gelagertes, an seinem Freiende den Stoßwellenkopf tragendes zweiten Bogensegment. Das erste Bogensegment und der Röntgen-C-Bogen selbst sind um eine in der Orbitalebene und durch das Isozentrum des Röntgen-C-Bogens verlaufende gemeinsame Horizontalachse, also angular, verschwenkbar. Aufgrund dieser Ausgestaltung lässt sich ein Stoßwellenkopf sowohl oberhalb als auch unterhalb eines Patiententisches positionieren. Nachteilig ist jedoch, dass der vom Röntgen-C-Bogen umgrenzte Raum durch die darin vorhandenen Tragvorrichtung derart beengt ist, dass eine horizontale und parallel zur Orbitalebene verlaufende Verschiebung des Patiententisches kaum noch möglich ist. Bei einem Wechsel von einer linksseitigen zu einer rechtsseitigen Behandlungsposition kann daher das jeweilige Patientenvolumen nicht durch eine Tischverschiebung bei unveränderter Patientenlage in den Fokuspunkt bzw. das Isozentrum gebracht werden. Es ist vielmehr eine Kopf-zu-Fuß-Umlagerung des Patienten erforderlich. Die Folge ist, dass die bisherige Rauminstallation der neuen Patientenlage angepasst werden, beispielsweise ein zeitaufwändiger Umbau von Zusatzgeräten wie Anästhesiegeräten vorgenommen werden muss. US-A-5 044 354 beschreibt eine Anlage gemäß dem Oberbegriff des ersten Anspruchs.

Aufgabe der Erfindung ist es, eine Anlage zur Stoßwellenbehandlung vorzuschlagen, die in dieser Hinsicht verbessert ist.

Diese Aufgabe wird durch eine Anlage nach Anspruch 1 gelöst. Diese umfasst einen orbital um ein Isozentrum verfahrbaren Röntgen-C-Bogen und eine zu diesem axial versetzt und ortsfest angeordnete Tragvorrichtung für den Stoßwellenkopf. Ein sich zum Röntgen-C-Bogen hin erstreckender Ausleger ist mit seinem Fixierende mit der Tragvorrichtung verbunden und trägt mit seinem Freiende den Stoßwellenkopf. Mit Hilfe der Tragvorrichtung ist der Ausleger derart beweglich geführt, dass der Stoßwellenkopf in der Orbitalebene innerhalb eines Winkelbereiches von mindestens 180° ober- und unterhalb eines Patiententisches beliebig positionierbar und auf das Isozentrum ausrichtbar ist. Durch die axial versetzte Anordnung der Tragvorrichtung ist der gesamte vom Röntgen-C-Bogen umschlossene Raum frei zugänglich. Dies erlaubt es, einen Patiententisch horizontal soweit zu verschieben, dass ein Behandlungswechsel von der rechten zur linken Patientenseite, also eine Positionierung des links- oder rechtsseitigen Behandlungsgebietes des Patienten im Isozentrum, erfolgen kann, ohne eine Kopf-zu-Fuß-Umlagerung des Patienten vornehmen zu müssen. Dadurch kann das ursprüngliche Setup der Anlage erhalten bleiben, was insbesondere dann vorteilhaft ist, wenn etwa ein Patient mit beidseitigen Nierensteinen behandelt wird. Aufgrund der Beweglichkeit des Auslegers und damit des Stoßwellenkopfes in einem Winkelbereich von mindestens 180° lässt sich letzterer beispielsweise in einer Untertischposition mit vertikaler Ausrichtung seiner Stoßwellenachse (0°-Position) und in einer Obertischposition mit gleicher Stoßwellenachsenausrichtung (180°-Position) anordnen. Bei einem Verschwenkbereich von 230° kann eine Verschwenkung von der vertikalen Obertischposition (180°) bis zu einer unter dem Tisch durchgeschwenkten -50°-Position erfolgen. Hierdurch sind nahezu alle Behandlungssituationen an einem Patienten in ein und derselben Patientenlage durchführbar. Im Extremfall kann die Tragvorrichtung so ausgestaltet sein, dass mit dem Stoßwellenkopf ein Winkelbereich von 360° abgedeckt werden kann. Es steht also eine große Variabilität hinsichtlich der Wahl der Behandlungsposition des Stoßwellenkopfs zur Verfügung, beispielsweise kann eine Ureterstein-Behandlung von einer Ober- oder Untertischposition aus bei Rückenlage des Patienten vorgenommen werden.

Ist die Tragvorrichtung für den Stoßwellenkopf bezüglich des Röntgen-C-Bogens in Kopfrichtung des Patienten angeordnet, so hat der Arzt bis auf Höhe der zu behandelnden Stelle des Patienten auch auf der maschinenzugewandten Seite vom Fußbereich des Patienten her freien Zugang zu diesem, so dass etwa ein transuretraler Eingriff behinderungsfrei möglich ist. Trotz der genannten Anordnung ist für einen im Kopfbereich des Patienten arbeitenden Anästhesist noch genügend Bewegungsfreiheit vorhanden.

Aufgrund der orbitalen Verfahrbarkeit des Röntgen-C-Bogens kann sowohl die Ortung als auch die Beobachtung während der Behandlung, etwa der Fortgang einer Steinzertrümmerung, aus Richtung der Stoßwellenachse erfolgen, was eine höhere Zielgenauigkeit bietet (Inline-Ortung). Eine Abschattung des Röntgenstrahls durch eine innerhalb des Röntgen-C-Bogens angeordnete Tragstruktur für den Stoßwellenkopf ist dabei nicht zu befürchten. Innerhalb des vom Röntgenstrahl des Röntgensystems überstrichenen Volumenbereichs ist lediglich der Stoßwellenkopf selbst angeordnet. Der ihn tragende Ausleger stört dabei nicht, insbesondere wenn dieser mit seinem Freiende den Stoßwellenkopf von der Seite her fasst. Zusammenfassend steht erfindungsgemäß somit eine Anlage zur Verfügung, die eine Stoßwellenbehandlung in beliebigen Winkelpositionen sowie aus unterschiedlichen Einschallwinkeln bei stets gleichbleibender Ausrichtung und Rückenlage des Patienten, sowie die zielgenaue Röntgen-Inline-Ortung und eine nahezu behinderungsfreie Beobachtung mit Hilfe des Röntgensystems während der Behandlung gestattet. Die Anlage ist daher für eine Vielzahl von Anwendungen, beispielsweise der IPP, Nieren-, Ureter- und Blasensteine, transuretrale Eingriffe gleichermaßen geeignet.

Dadurch, dass beide Teilsysteme, nämlich das Röntgensystem und das Therapiesystem ortsfest zueinander, bspw. an einem gemeinsamen Grundkörper gebracht sind, ist deren relative Position zueinander mechanisch festgelegt. So kann etwa bei der Montage der Anlage eine Justierung dahingehend erfolgen, dass der Fokuspunkt des Stoßwellenkopfes in jeder Behandlungsposition auf das Isozentrum gerichtet ist bzw. mit diesem zusammen fällt. Der Einsatz etwa eines elektronischen Ortungssystems zur Positionsfeststellung bzw. Berechnung der Lage von Fokus- und Isozentrum ist daher nicht erforderlich.

Der Ausleger ist in einer zur Orbitalebene des Röntgen-C-Bogens parallelen Ebene zwangsgeführt. Ein seitliches Ausweichen des Fokuspunktes des Stoßwellenkopfes aus der Orbitalebene des Röntgen-C-Bogens ist dadurch verhindert.

Die Tragvorrichtung ist in zum Röntgen-C-Bogen axial versetzt und koaxial angeordneter C-Bogen (im weiteren als Therapiebogen bezeichnet), an dem der Ausleger mit seinem Fixierende orbital verfahrbar gelagert ist. Diese Ausgestaltung gestattet eine vollständig zwangsgeführte Bewegung des Stoßwellenkopfes in der Orbitalebene des Röntgen-C-Bogens. Eine bei der Neuinstallation einer Anlage vorgenommene Justierung des Fokuspunktes des Stoßwellenkopfes auf das Isozentrum des Röntgen-C-Bogens bleibt erhalten.

Eine Verfahrung des Stoßwellenkopfes um einen bestimmten Winkelbereich setzt im Nörmalfall einen Therapiebogen mit mindestens entsprechend bemessener Bogenlänge voraus. Bei einer Verfahrbarkeit des Stoßwellenkopfes bspw. um 250° würde ein entsprechend bemessener Therapiebogen einen Behandlungstisch ober- und unterseitig weit übergreifen und dadurch die Bewegungsfreiheit eines behandelnden Arztes auf der Behandlungsseite des Patiententisches einschränken. Um dies zu verhindern, weist eine bevorzugte Anlagenvariante einen Therapiebogen auf, der orbital verfahrbar gelagert ist. Der Therapiebogen kann nun wesentlich kürzer sein, da sich der maximale Verfahrweg des Stoßwellenkopfes aus der Verfahrstrecke des Therapiebogens einerseits und der Verfahrstrecke des Stoßwellenkopfes am Therapiebogen andererseits ergibt. Zur Verkürzung der Therapiebogenlänge ist es auch denkbar, dass dieser aus zwei gegeneinander orbital verschiebbaren Bogensegmenten gebildet ist. Eine andere Möglichkeit zur Bogenverkürzung besteht darin, den Ausleger so am Therapiebogen drehbar zu fixieren, dass sein Freiende in eine über ein Schenkelende des Therapiebogens hinausragende Position geschwenkt werden kann.

Bei einer zweiten Ausführungsform der Anlage die nicht zur Erfindung gehöst, ist die Tragvorrichtung ein mehrere über Gelenke verbundene Armsegmente aufweisender Gelenkarm, mit dessen Freiende das Fixierende des Auslegers verbunden ist. Während mit einem Therapiebogen eine Festlegung der Bewegung des Auslegers bzw. des Stoßwellenkopfes auf einer Kreisbahn verbunden ist, können die gewünschten Behandlungspositionen des Stoßwellenkopfes bei Verwendung eines Gelenkarms als Tragvorrichtung mit beliebigen Bewegungsbahnen angefahren werden, wobei dann eine Steuereinrichtung zur isozentrischen Ausrichtung des Stoßwellenkopfes erforderlich ist. Bei einer vorteilhaften Ausgestaltung ist der Freiheitsgrad des Gelenkarmes so eingeschränkt, dass er sich nur innerhalb einer zur Orbitalebene des Röntgen-C-Bogens parallelen Ebene bewegen kann. Dies wird zweckmäßiger Weise dadurch erreicht, dass die die Armsegmente verbindenden Gelenke des Gelenkarmes parallel zueinander und rechtwinklig zur Orbitalebene des Röntgen-C-Bogens verlaufende Drehachsen aufweisen, also all Scharniergelenke ausgebildet sind. Um den Stoßwellenkopf in jeder Winkelposition isozentrisch ausrichten zu können, ist der Ausleger drehbar mit dem Freiende des Gelenkarms verbunden.

Bei beiden Ausführungsformen ist ein Stoßwellenkopf vorgesehen, der von einem für Röntgenstrahlen durchlässigen, sich längs seiner Stoßwellenachse erstreckenden Zentralbereich durchsetzt ist. Diese Ausgestaltung lässt eine zielgenaue "Inline-Ortung" mit dem Röntgensystem ohne Lageveränderung des Stoßwellenkopfes, also auch während einer Lithotripsiebehandlung zu. Bei einer ebenfalls für beide Ausführungsformen vorteilhaften Ausgestaltung ist die Tragvorrichtung zusammen mit dem Stoßwellenkopf aus einer Behandlungsstellung in eine von einem Patiententisch bzw. einem darauf gelagerten Patienten entfernte Parkposition verfahrbar. Dadurch kann die Bewegungsfreiheit in dem sich zwischen Röntgen-C-Bogen und Kopfseite des Patiententisches befindlichen Raum bzw. allgemein im Abdominalbereich des Patienten erhöht werden.

Um eine Orbitalverfahrung des Röntgen-C-Bogens und des Therapiebogens bzw. eine Bewegung des Gelenkarms auf der Unterseite des Patiententisches nicht zu behindern, ist dieser endseitig, beispielsweise kopfseitig, also außerhalb des Bewegungsbereiches der genannten Vorrichtungen, gelagert.

Für eine weitere Beschreibung der Erfindung wird auf die Ausführungsbeispiele der Zeichnungen verwiesen. Es zeigen, jeweils in einer perspektivischen Prinzipdarstellung:
Fig. 1 eine Stoßwellen-Lithotripsie-Anlage in einer ersten Ausführungsform mit Stoßwellenkopf in Obertisch-Behandlungsposition und Röntgen-C-Bogen in Grundposition (Inline zum Stoßwellenkopf),
Fig. 2 die Anlage aus Fig. 1 mit Stoßwellenkopf in Untertisch-Behandlungsposition für die linke (maschinenferne) Patientenseite mit orbital in Inline-Position verschwenktem Röntgensystem,
Fig. 3 die Anlage aus Fig. 1 mit Therapie-C-Bogen und Stoßwellenkopf in Parkposition.
Fig. 4 die Stoßwellen-Lithotripsie-Anlage in einer zweiten Ausführungsform die nicht zur Erfindung gehöst, mit Gelenkarm und Stoßkopf in Parkposition und Röntgen-C-Bogen in Grundposition,
Fig. 5 die SWL-Anlage aus Fig. 4 mit geparktem, also ausgeschwenktem Röntgen-C-Bogen und Stoßkopf in Behandlungsposition (Untertisch - rechts),
Fig. 6 die Anlage aus Fig. 4 mit Stoßkopf in Behandlungsposition (Untertisch - links) und gekipptem Röntgen-C-Bogen in Inline-Position.

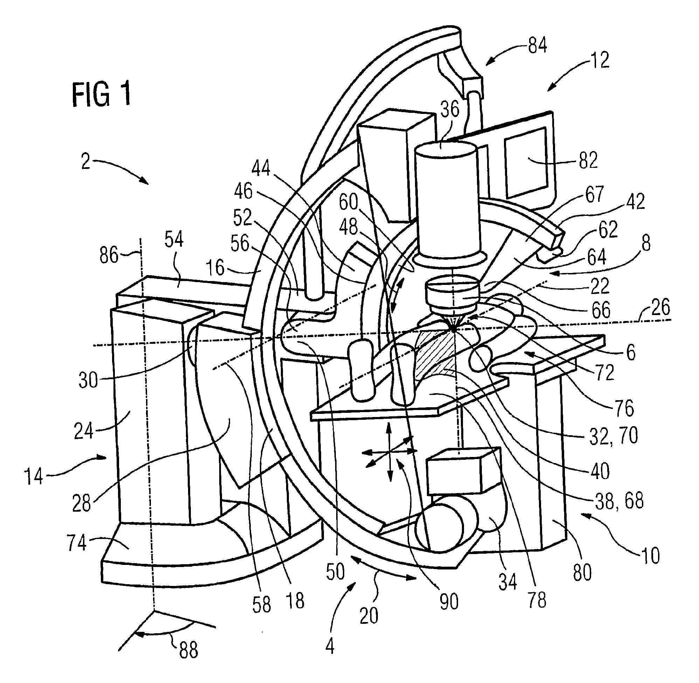

Fig. 1 zeigt eine SWL-Anlage 2 in einer ersten Ausführungsform, die folgende Teilkomponenten umfasst: einen Röntgen-C-Bogen 4, einen einen Stoßwellenkopf 6 tragenden, die erste Ausführungsform charakterisierenden Therapie-C-Bogen 8 als Tragvorrichtung, einen Patiententisch 10 und ein Anzeigemodul 12. Der Röntgen-C-Bogen 4 umfasst einen zweiteiligen Grundkörper 14, an dem ein C-Bogen-Segment 16 verfahrbar gelagert ist. Hierzu ist im Grundkörper 14 ein kreissegmentförmiges Lager 18 (nicht sichtbar) vorhanden, in dem das C-Bogen-Segment 16 möglichst spielfrei zwangsgeführt ist. Das C-Bogen-Segment 16 kann deshalb im Lager 18 in der durch den Doppelpfeil 20 angedeuteten Orbitalrichtung eindimensional bewegt werden.

Der zweiteilige Grundkörper 14 umfasst einen Sockel 24, der ortsfest ruht. An diesem ist, um eine waagerecht verlaufende Schwenkachse 26 drehbar, eine Führung 28 über ein Drehgelenk 30 angebracht. Die Schwenkachse 26 schneidet eine Längsachse 22 in einem Isozentrum 32. Um die Schwenkachse 26 ist der Röntgen-C-Bogen 4 angular verschwenkbar. Die orbitale Verschwenkbewegung des C-Bogen-Segments 16 erfolgt bei der in Fig. 1 gezeigten angularen Grundposition um eine waagerecht verlaufende Längsachse 22. Bei angularer Verschwenkung des Röntgen-C-Bogens 4 erfolgt dann dessen orbitale Verschwenkung um eine entsprechend zur Längsachse 22 gekippte Drehachse (nicht dargestellt).

An den beiden Enden des C-Bogen-Segments 16 sind eine Röntgenquelle 34 und ein Bildverstärker 36 montiert. Die Röntgenquelle 34 und der Bildverstärker 36 bilden zusammen ein Bildgebungssystem, dessen Zentralstrahl 38 ebenfalls durch das Isozentrum 32 verläuft. So ist sichergestellt, dass der Zentralstrahl 38 in jeder Angular- und Orbitalposition des C-Bogen-Segments 16 das Isozentrum 32 durchstößt.

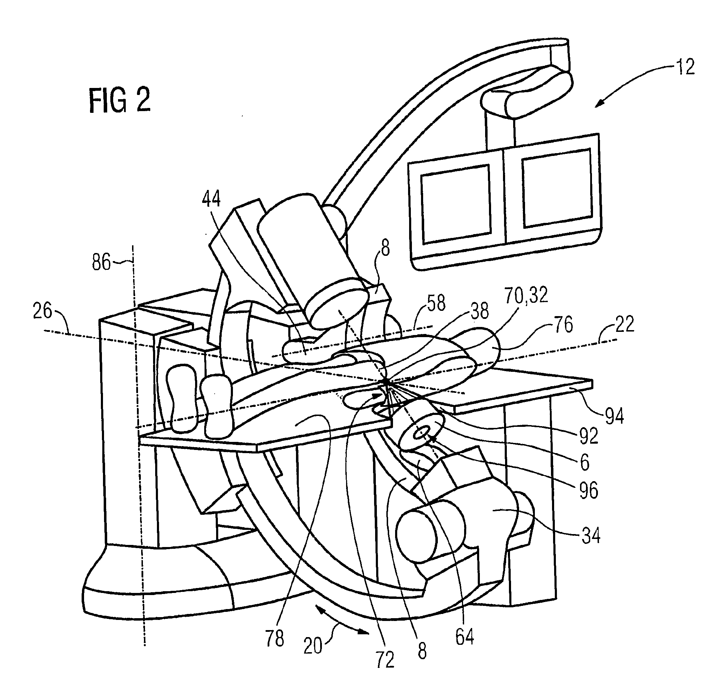

In Fig. 1 ist das C-Bogen-Segment 16 in seiner Grundposition dargestellt, d.h. der Zentralstrahl 38 verläuft in senkrechter Richtung. Durch orbitales Verfahren des C-Bogen-Segments 16 in Richtung 20 (wie z.B. in Fig. 2 dargestellt), spannt der mitwandernde Zentralstrahl 38 eine Orbitalebene 40 auf, welche den Zentralstrahl 38 und die Schwenkachse 26 beinhaltet. Die Orbitalebene 40 ist in Fig. 1 aus Gründen der Übersichtlichkeit nur in einem sehr kleinen Bereich schraffiert dargestellt, erstreckt sich jedoch auch über den schraffierten Bereich und den Durchmesser des Röntgen-C-Bogens 4 hinaus.

Der Therapie-C-Bogen 8 ist an seiner radial außen liegenden Seite 42 an einer Führung 44 gelagert. In der Führung 44 ist hierzu ein dem Lager 18 entsprechendes, nicht sichtbares Lager 46 vorhanden, an dem der Therapie-C-Bogen 8 in Richtung des Pfeils 48 orbital verfahrbar ist. Mit ihrem Ende 50 ist die Führung 44 am Lagerbock 52 eines Grundkörpers 54 gelagert. Hierbei ist ein nicht sichtbares Gelenk 56 zwischen Lagerbock 52 und Führung 44 angeordnet, welches eine Drehung um eine zur Längsachse 22 parallele Achse 58 erlaubt.

Alternativ oder zusätzlich zur dargestellten Ausführungsform können auch in den Figuren nicht dargestellte Führungsschienen mit entsprechenden Schlitten am Grundkörper 54 bzw. Lagerbock 52 und an der Führung 44 angebracht sein, auf denen der Therapie-C-Bogen 8 zusammen mit der Führung 44 z.B. parallel zur Achse 26 aus dem Patientenbereich wegschiebbar ist. Es sind auch andere Anordnungen von Schienen denkbar, so dass der Röntgen-C-Bogen 4 zusammen mit dem Stoßwellenkopf 6 entlang dieser in gewissen Grenzen zweidimensional bewegbar ist.

An der radial innen liegenden Seite 60 des Therapie-C-Bogens 8 ist ein Schlitten 62 ebenfalls in Richtung 48 orbital verfahrbar gelagert. Am Schlitten 62 ist ein Ausleger 64 mit seinem Fixierende 67 befestigt, der in Richtung zum Röntgen-C-Bogen 4 hin weist und an seinem Freiende 66 den Stoßwellenkopf 6 trägt. Zum orbitalen Verfahren des Stoßkopfes 6 werden Schlitten 62 am Therapie-C-Bogen 8 und Therapie-C-Bogen 8 an der Führung 44 gleichzeitig verfahren, z.B. über einen im Inneren des Therapie-C-Bogens 8 angeordneten, in Fig. 1 nicht sichtbaren Kettenantrieb. Dass dabei die beiden eben genannten Bewegungen nicht mehr unabhängig voneinander sind, ist für die Funktionalität der Anlage 2 unerheblich. Die Orbitalbewegungen von Therapie-C-Bogen 8 und Schlitten 62 erfolgen ebenfalls um die Längsachse 22.

Der gesamte Therapie-C-Bogen 8 mit seinem Grundkörper 54 ist um einen Axialabstand zum Röntgen-C-Bogen 4 bzw. zur Orbitalebene 40 parallel versetzt, das heißt, die Ebene, die der Therapie-C-Bogen 8 aufspannt, liegt parallel zur Orbitalebene 40 und zu dieser beabstandet. Der Ausleger 64 erstreckt sich in Richtung auf den Röntgen-C-Bogen 4 soweit hin, dass der an ihm befestigte Stoßwellenkopf 6 wiederum in der Orbitalebene 40 liegt. Der Abstand ist derart bemessen, dass ein Fokuspunkt 70 einer vom Stoßwellenkopf 6 ausgesandten, in Fig. 1 durch den Kegel 72 dargestellten Ultraschallstoßwelle in der Orbitalebene 40 liegt, wobei die Kegelspitze den Fokuspunkt 70 bildet und im Isozentrum 32 liegt. Der Stoßwellenkopf 6 ist ein Ultraschall-Stoßwellenkopf zur Erzeugung eines im Fokuspunkt 70 fokussierten Ultraschallimpulses.

Die Stoßachse 68, also die Ausbreitungsrichtung des Ultraschallpulses, führt hierbei durch den Fokuspunkt 70, liegt in der Orbitalebene 40 und fällt in Fig. 1 mit dem Zentralstrahl 38 zusammen. Aus diesem Grund spricht man in Fig. 1 von einer sogenannten Inline-Position von Stoßwellenkopf 6 und Röntgensystem 34, 36. Durch eine die Stoßachse 68 umgebende röntgentransparente Zone 96 (in Fig. 2 sichtbar) im Stoßwellenkopf 6 kann nämlich während der Stoßwellenbehandlung eines Patienten 76 eine gleichzeitige Röntgenortung des zu behandelnden Objekts bzw. eine Durchleuchtung der Umgebung des Fokuspunktes 70 im Inneren des Patientenkörpers stattfinden. Die von der Röntgenquelle 34 ausgesandten Röntgenstrahlen können die röntgentransparente Zone 96 des Stoßwellenkopfes 6 entlang des Zentralstrahls 38 durchdringen. Gleichzeitig ist der Stoßwellenkopf 6 auf der Bauchseite des Patienten positioniert, um z.B. einen Stein im Harnleiter des Patienten zu behandeln. Hier spricht man von der sogenannten Obertisch-Behandlungsposition.

Durch die koaxiale Anordnung von Röntgen-C-Bogen 4 und Therapie-C-Bogen 8 bleibt die Lage des Fokuspunktes 70 im Isozentrum 32 in jeder Verfahrposition des Stoßwellenkopfes 6 erhalten. Die Stoßachse 68 liegt immer in der Orbitalebene 40.

Die exakte geometrische Ausrichtung von Gelenkarm 8 und Röntgen-C-Bogen 4 zueinander erfolgt dadurch, dass der Grundkörper 14 und der Grundkörper 54 auf einem gemeinsamen Fußteil 74 montiert sind. Die Ausrichtung wird hierbei bei der Herstellung der SWL-Anlage 2 im Werk vorgenommen.

Der Behandlungspunkt der SWL-Anlage 2, in dem der Fokuspunkt 70 zu lazieren ist, liegt immer im Isozentrum 32. In einer Bildgebungsphase der Behandlung des Patienten 76 wird dieser deshalb mit seiner zu behandelnden Stelle ins Isozentrum 32 gebracht (In Fig. 1 bereits geschehen). Um die zu behandelnde Stelle im Inneren des Patienten 76 nichtinvasiv zu orten, liefert das Bildgebungssystem, bestehend aus Röntgenquelle 34 und Bildverstärker 36 Röntgenaufnahmen, welche auf Bildschirmen 82 des Anzeigemoduls 12 dargestellt werden. Über den flexiblen, gewichtsausgeglichenen Tragarm 84 kann der Bildschirm 82 in eine günstige Betrachtungsposition für das Bedienpersonal der Anlage 2 verfahren werden. Um den Behandlungspunkt dreidimensional zu orten, werden mindestens zwei Röntgenbilder des Patienten 76, eventuell bei zunächst weggeschwenktem Stoßwellenkopf 6 erstellt, in dem der Röntgen-C-Bogen 4 um die Achsen 22 (orbitale Ortung) oder 26 (angulare Ortung) z.B. zwischen den in Fig.1 und Fig.2 dargestellten Positionen verschwenkt wird. Zum Verfahren des Patienten 76 ist eine Liegefläche 78, auf der der Patient 76 ruht, an einem fest installierten Sockel 80 kopfgelagert und in sämtliche Raumrichtungen 90 linear verfahrbar.

Das Heranführen des Stoßwellenkopfes 6 an den Patienten 76 kann auf zwei Arten erfolgen. Entweder wird die Behandlungsposition des Patienten 76 zuerst aufgesucht und dann markiert, z.B. bei einem motorisch verfahrbaren Liegefläche 78 elektronisch gespeichert. Die Liegefläche 78 zusammen mit dem Patienten 76 wird danach ein Stück verfahren, damit der Stoßwellenkopf 6 in die in Fig. 1 gezeigte Position verfahren werden kann, dann wird der Patient 76 an den Stoßwellenkopf 8 von unten her herangefahren, bis die oben gespeicherte Behandlungsposition wieder erreicht ist. Somit ist die in Fig. 1 dargestellte Position hergestellt.

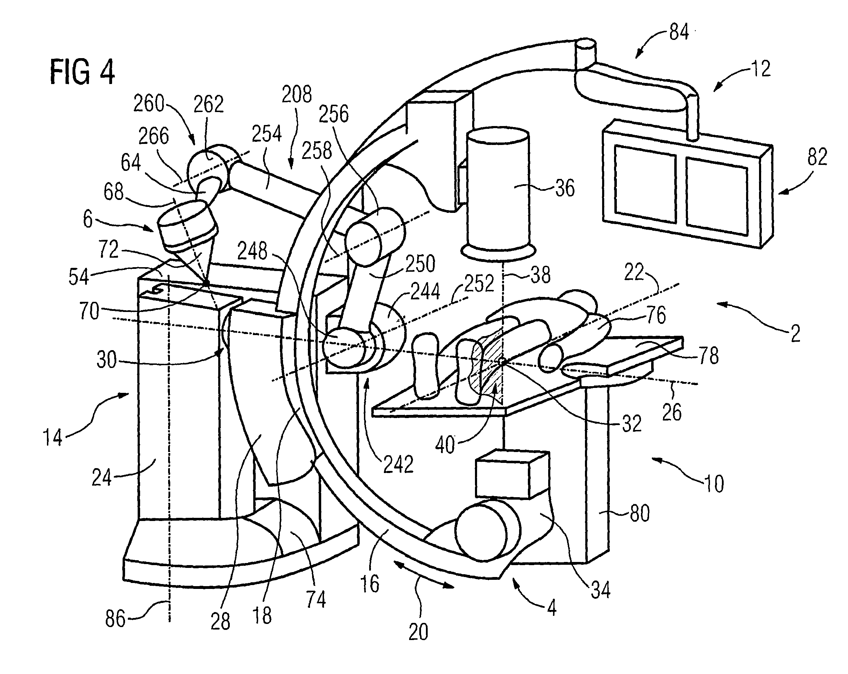

Alternativ kann wegen des um die Achse 58 schwenkbaren gesamten Therapie-C-Bogens 8 die Ankopplung des Stoßwellenkopfes 6 auch an den in Behandlungsposition gebrachten und von nun an ruhenden Patienten 76 erfolgen, indem der vorher nach oben geschwenkte Therapie-C-Bogen 8 zusammen mit dem Stoßwellenkopf 6 auf den nach oben weisenden Bauch des Patienten 76 abgesenkt wird. Diese Ankoppelvariante gilt insbesondere für die Ausführungsform der SWL-Anlage 2 gemäß Fig.4 bis Fig.6.

Ist der Stoßwellenkopf 6 an den Patienten 76 angekoppelt, kann mit der Behandlung durch Einschalten der Ultraschallstoßwelle begonnen werden.

Um eine weitere Drehachse 86, die senkrecht den Grundkörper 14 und das Fußteil 74 durchsetzt, ist der gesamte Röntgen-C-Bogen 4 aus dem Patientenbereich weg schwenkbar (in den Figuren nicht dargestellt), wenn er gerade nicht benötigt wird, was den Zugang für das Behandlungspersonal zum Patienten 76 erhöht. Das Verschwenken erfolgt aus der in Fig. 1 dargestellten Grundposition in Richtung des Pfeils 88.

Ein Ausweichen des Stoßwellenkopfes 6, bedingt durch sein Eigengewicht und den Anpressdruck am Patienten und die Verformung des Therapie-C-Bogens 8 kann z.B. durch eine leichte Verschwenkung des Therapie-C-Bogens 8 um die Achse 58 korrigiert werden.

Die in Fig. 2 dargestellte Behandlungsposition, nämlich die sogenannte Untertisch-Links-Position behandelt beispielsweise die linke Niere des Patienten 76. Der Stoßwellenkopf 6 ist in Untertischposition verschwenkt. Der in Fig. 2 von der Röntgenquelle 34 verdeckte Schlitten 62 ist gegenüber Fig. 1 an das entgegengesetzte Ende des Therapie-C-Bogens 8 verfahren. Zusätzlich ist der Therapie-C-Bogen 8 selbst in seiner Führung 44 an das gegenüber Fig. 1 entgegengesetzte Ende verfahren. Der Stoßwellenkopf 6 ragt in eine Aussparung 92 der Liegefläche 78, um möglichst nah zum direkten Kontakt an den Patienten 76 heran gebracht zu werden. Der Kegel 72 der vom Stoßwellenkopf 6 erzeugten Ultraschallstrahlen durchsetzt hierbei einen nicht dargestellten, wassergefüllten Koppelbalg, welcher zwischen Stoßwellenkopf 6 und Patient 76 unter Zwischenlage von Gel angekoppelt ist und weiter das Körpergewebe des Patienten soweit, dass der Fokuspunkt 70 das Zentrum eines nicht dargestellten Nierensteins im Körper des Patienten 76 trifft.

Bezüglich der Achse 26 befindet sich der Röntgen-C-Bogen wie in Fig. 1 in Grundposition. Allerdings ist er in Richtung 20 im Gegenuhrzeigersinn um ca. 40 Grad verschwenkt, um den Patienten 76 schräg zu durchleuchten. Die 40-Grad-Position ist eine übliche Position zur Behandlung von Nierensteinen.

In Fig. 2 ist zu erkennen, daß der Stoßwellenkopf 6 am Ausleger 64 exzentrisch angebracht ist, nämlich an der in Untertischposition anlagenabgewandten Seite des Auslegers 64. Hierdurch steht der Stoßwellenkopf 6 in Richtung Vorderseite 94 der Liegefläche weiter vom Patiententisch 10 vor als der Therapie-C-Bogen 8 und der Ausleger 64. Der in der Regel an der Vorderseite 94 neben dem Patiententisch 10 stehende Arzt ist hierdurch so wenig wie möglich in seiner Bein- bzw. Bewegungsfreiheit eingeschränkt. Da in Obertischposition die in Fig. 1 gezeigte 180-Grad Position des Stoßwellenkopfes 6 die äußerste Position des Stoßwellenkopfes 6 ist, ist auch hier der Überstand von Therapie-C-Bogen 8 und Ausleger 64 für den behandelnden Arzt in dessen Kopfbereich tragbar. Außerdem steht hierdurch eine weitere Möglichkeit zu Verfügung, den C-Bogen zu verkleinern.

In Fig. 2 ist die zentrale röntgentransparente Zone 96 im Stoßwellenkopf 6 sichtbar, die zur Inline-Ortung bei der Stoßwellenbehandlung dient. Wegen des ortsfest ruhenden Isozentrums 32 ist die Liegefläche 78 gegenüber Fig. 1 etwas erhöht und zur rechten Patientenseite hin verschoben, um statt dessen Harnleiter dessen linke Niere im Behandlungspunkt, also dem Isozentrum 32 zu platzieren.

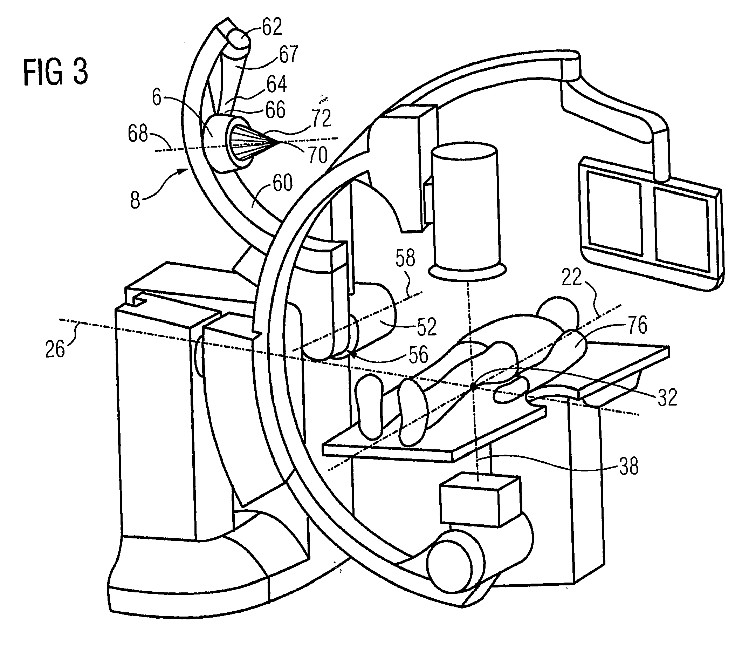

Fig. 3 zeigt den Therapie-C-Bogen 8 in Parkposition. Der gesamte Therapie-C-Bogen 8 zusammen mit dem Stoßwellenkopf 6 ist aus der in Fig. 1 gezeigten Position um die Achse 58 nach oben um ca. 90 Grad verschwenkt. Hierdurch wird der gesamte Patientenoberkörperbereich freigegeben, was den Zugang für Behandlungspersonal zum Patienten 76 deutlich erleichtert. Dies ist in einer Notsituation oder bei der Behandlungsvoroder -nachbereitung von Vorteil.

Fig. 4 zeigt die SWL-Anlage 2 in einer alternativen Ausführungsform die nicht zur Erfindung gehöst, nämlich mit einem Gelenkarm 208 als Tragvorrichtung.

Der Gelenkarm 208 ist mit seinem einen Ende 242 am Lagerbock 244 des Grundkörpers 54 gelagert. Hierbei ist ein Gelenk 248 zwischen Lagerbock 244 und einem Armsegment 250 angeordnet, welches eine Drehung um eine zur Längsachse 22 parallele Achse 252 erlaubt. Zwischen dem Armsegment 250 und einem weiteren Armsegment 254 ist ein weiteres Gelenk 256 angebracht, welches um eine ebenfalls parallel zur Längsachse 22 verlaufende Achse 258 schwenkbar ist. Am Freiende 260 des Gelenkarms 208 ist ein weiteres Gelenk 262 angebracht, welches das Armsegment 254 mit dem Ausleger 64 verbindet und dessen Drehung zusammen mit dem Stoßkopf 6 um die ebenfalls parallel zur Längsachse 22 verlaufende Achse 266 erlaubt.

Der gesamte Gelenkarm 208 mit seinem Grundkörper 46 ist um einen Axialabstand zum Röntgen-C-Bogen 4 bzw. zur Orbitalebene 40 parallel versetzt, das heißt, die Längsachsen der Armsegmente 250 und 254 verlaufen parallel zur Orbitalebene 40. Der Ausleger 64 erstreckt sich in Richtung auf den Röntgen-C-Bogen 4 soweit hin, dass der an ihm befestigte Stoßkopf 6 wiederum in der Orbitalebene 40 liegt. Der Abstand ist derart bemessen, dass der Fokuspunkt 70 der vom Stoßkopf 6 ausgesandten, in Fig. 4 durch den Kegel 72 dargestellten Ultraschallstoßwelle in der Orbitalebene 40 liegt. Die Stoßachse 68 führt wieder durch den Fokuspunkt 70 und liegt in der Orbitalebene 40.

Durch die Parallelität sämtlicher Achsen 252, 258 und 266, um welche die Einzelteile des Gelenkarms 208 schwenkbar sind, ist der Fokuspunkt 70 lediglich zweidimensional verschiebbar und zwar stets innerhalb eines durch die Abmessungen des Gelenkarms 208 begrenzten Bereiches der Orbitalebene 40. Insbesondere kann der Fokuspunkt 70 durch Verschwenken des Gelenkarms 208 zum Isozentrum 32 geführt werden.

In Fig. 4 ist der Gelenkarm 208 und der Stoßkopf 6 in eine sogenannte Parkposition verfahren, d.h. möglichst weit aus dem Umgebungsbereich des auf dem Patiententisch 10 ruhenden Patienten 76 herausgefahren. Der Zugang zum Patienten 76 von allen Seiten für nicht dargestelltes Behandlungspersonal bzw. Ärzte ist somit behinderungsfrei möglich. In der in Fig. 4 dargestellten Situation ist kann z.B. eine Bildgebungsphase vor oder nach der Behandlung des Patienten 76 erfolgen. In Fig. 4 ist zwar der Ultraschallkegel 72 und der Fokuspunkt 70 dargestellt, jedoch die Ultraschallquelle in der Regel ausgeschaltet.

Liegt der zu behandelnde Körperbereich des Patienten 76 im Isozentrum 32, so wird der Stoßkopf 6 durch Verschwenken des Gelenkarms 208 zum Patienten hin geführt. Der Patient muss hierzu nicht erneut bewegt werden. Der Röntgen-C-Bogen 2 ist hierbei um die Schwenkachse 26 kurzfristig aus seiner in Fig. 4 gezeigten angularen Grundposition wegzukippen, um eine Kollision zu vermeiden. Ist der Fokuspunkt 70 ins Isozentrum 32 verbracht, kann mit der Behandlung durch Einschalten der Ultraschallstoßwelle begonnen werden.

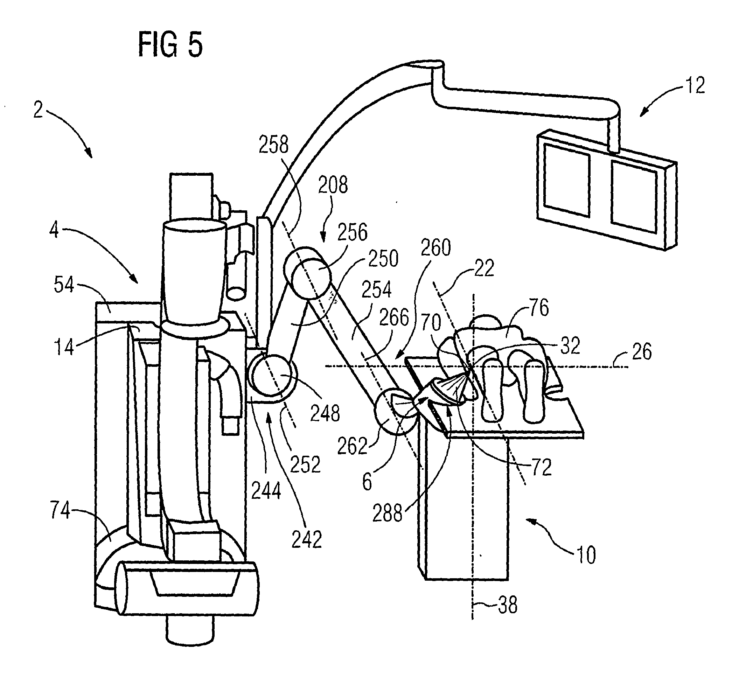

Um die Drehachse 86, die senkrecht den Grundkörper 14 und das Fußteil 74 durchsetzt, ist der gesamte Röntgen-C-Bogen 4 aus dem Patientenbereich weg schwenkbar, was den uneingeschränkten Zugang für das Behandlungspersonal zum Patienten 76 ermöglicht. Diese Parkposition des Röntgen-C-Bogens 4 ist in Fig. 5 dargestellt. Der Gelenkarm 208 ist statt dessen in eine Behandlungsposition verfahren, bei der der Fokuspunkt 70 mit dem Isozentrum 32 zusammenfällt. So kann die rechte Niere aus ca. 40°-Position rückenwärts des Patienten 76 behandelt werden - dies ist die sogenannte Untertisch-rechts-Position.

In den Gelenken 248, 256 und 262 sind nicht dargestellte Winkelaufnehmer vorhanden, welche die jeweilige Drehposition des betreffenden Gelenks erfassen und an einen nicht dargestellten Zentralrechner weiterleiten. Die jeweilige Position der Armsegmente 250 und 252 bzw. des Stoßwellenkopfes 6 und somit des Fokuspunkts 70 kann aus den bekannten Abmessungen des gesamten Gelenkarms 208 durch Erfassung der Drehwinkel der Gelenke 248, 256 und 262 auf geeignete Weise im Zentralrechner ermittelt werden. Dieser steuert die ebenfalls nicht dargestellten Motoren in den Gelenken 248, 256 und 262 derart an, dass der Fokuspunkt 70 exakt im Isozentrum 32 zu liegen kommt. Eine automatisierte Steuerung des gesamten Gelenkarms 208 bzw. dessen Bewegung ist so ermöglicht.

Auf Grund des weg geschwenkten Röntgen-C-Bogens 4 sind die Längsachse 22, Schwenkachse 26 und Zentralstrahl 38 aus Fig. 4 nochmals gestrichelt eingezeichnet. Auf Grund der platzsparenden Anordnung des Gelenkarms 208 auf nur einer Seite des Patienten 76, nämlich der rechten, welche in Fig. 5 auch Behandlungsseite ist, ist der Zugang zum Patienten mit größtmöglichem Freiraum ermöglicht. Der Stoßkopf 6 ragt hierbei in eine der Aussparung 92 gegenüberliegende Aussparung 288 der Liegefläche 78, um wieder möglichst nah zum direkten Kontakt an den Patienten 76 heran gebracht zu werden.

Der Röntgen-C-Bogen 4 ist um die parallel zur Orbitalebene 40 und senkrecht zu den Drehachsen 252, 258, und 266 verlaufende Drehachse 86 (in Fig. 5 verdeckt) schwenkbar. Da die Drehachsen 252, 258, und 266 üblicherweise waagerecht verlaufen, die Orbitalebene 40 senkrecht steht, steht die Drehachse 86 für den Röntgen-C-Bogen 2 üblicherweise ebenfalls senkrecht. Der Röntgen-C-Bogen 2 kann somit nach Art der Bewegung einer Türe aus dem Behandlungsgebiet weg geschwenkt werden, wenn er nicht benötigt wird. Trotz weggeschwenktem Röntgen-C-Bogen 4 bleibt die Behandlung des Patienten 76 mit dem Stoßwellenkopf 6 ortsgenau, da sich deren Ortsposition zur SWL-Anlage 2 dadurch nicht verändert.

In einer derartigen Position der SWL-Anlage 2 ist dann eine Inline-Ultraschallortung möglich. Der Zugang zum Patienten 76 ist dann nämlich auch von der maschinenzugewandten Rückseite des Patiententisches 10 möglich. Durch den zur Kopfseite des Patienten 76 hin versetzten Gelenkarm 208 ist die Rückseite des Stoßwellenkopfes 6 frei zugänglich. So kann in eine nicht dargestellte zentrale Öffnung im Stoßwellenkopf 6 ein nicht dargestellter Ultraschall-Applikator eingesetzt werden und hierdurch eine Ultraschallortung des zu behandelnden Objekts im Patientenkörper durchgeführt werden. Die zentrale Öffnung ist etwa im Bereich der röntgentransparenten Zone 96 angeordent.

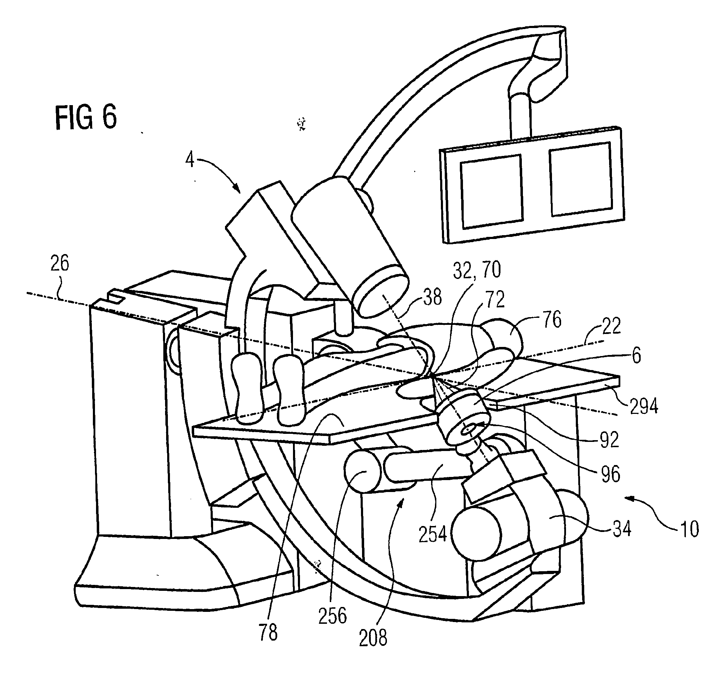

Fig. 6 zeigt eine Betriebssituation der Anlage 2, in der gleichzeitig zur Stoßwellenbehandlung des Patienten 76 mit Hilfe des Stoßkopfes 6 eine Röntgendurchleuchtung mit Hilfe des Röntgen-C-Bogens 4 erfolgt. Der Stoßwellenkopf 6 befindet sich in der Inline-Position. Die von der Röntgenquelle 34 ausgesandten Röntgenstrahlen können durch die röntgentransparente Zone 96 entlang des Zentralstrahls 38 den Stoßwellenkopf 6 durchdringen. Gleichzeitig ist der Stoßwellenkopf 6 auf der linken Patientenseite entsprechend zu Fig. 5, also in ca. -40°-Position positioniert, um z.B. einen Nierenstein der linken Niere des Patienten zu behandeln (Untertisch-Links-Position wie in Fig.2). Wegen des ortsfest ruhenden Isozentrums 32 ist die Liegefläche 78 gegenüber Fig. 5 etwa um den Abstand der Nieren des Patienten zur rechten Patientenseite hin verschoben. Der Fokuspunkt 70 fällt wieder mit dem Isozentrum 32 zusammen. Die Röntgenanordnung ist um die Längsachse 22 gekippt, um den Patienten 76 schräg zu durchleuchten. Die Aussparung 92 in der Liegefläche 78 bietet wiederum Platz für den Stoßkopf 6.

Aus Fig. 6 wird deutlich, dass obwohl sich der Stoßkopf 6 auf der gerätefernen Seite des Patiententisches 10 befindet, dieser kaum über die geräteferne Tischkante 294 hinausragt und somit den behandelnden Arzt genügend Beinfreiheit gibt und damit weiterhin größtmöglichen Patientenzugang erlaubt. Im Gegensatz zum ersten Ausführungform ist bei einem Gelenkarm 208 als Tragvorrichtung auch in Obertischposition (in den Figuren nicht gezeigt) außer dem Stoßwellenkopf 6 kein weiteres Bauteil im Kopf- oder Fußbereich des Arztes störend vorhanden.

Befinden sich Patient 76 und Liegefläche 78 sich in einer seitlichen Mittenposition zwischen den in Fig. 5 und 6 dargestellten Positionen, dann ist die dritte wesentliche, nicht in den Figuren dargestellte Möglichkeit gegeben, den Patienten 76 zu behandeln. Bei gegenüber Fig. 5 und 6 etwas abgesenkter Patientenlage ist der Stoßkopf 6 in Obertischposition verfahrbar, um den Patienten 76 von oben her, also dessen Bauchseite zentral im Harnleiterbereich zu behandeln. Der Stoßwellenkopf 6 wäre dann z.B. in Fig. 4 auf der Bauchoberseite des Patienten 76 zwischen diesem und dem Bildverstärker 36 angeordnet, so dass auch gleichzeitig wieder eine Röntgendruchleuchtung (Inline) des Patienten 76 stattfinden kann. Auch hier ragt kein Bauteil der Anlage 2 über die Tischkante 294 zur maschinenfernen Seite, an der der Arzt sich aufhält, hinaus. Ausgehend von der Position in Fig. 3 kann dies durch Kippen des Armsegments 254 um die Achse 258 und Kippen des Stoßkopfes 6 um die Achse 266 erfolgen.

## Patentansprüche

1. Anlage (2) zur bildgestützten Stoßwellenbehandlung, mit folgender Ausgestaltung:
- sie umfasst einen orbital um ein Isozentrum (32) verfahrbaren Röntgen-C-Bogen (4) mit einem Röntgensystem (34,36), einen Stoßwellenkopf (6) und eine seitlich vom und ortsfest zum Röntgen-C-Bogen (4) angeordnete Tragvorrichtung für den Stoßwellenkopf (6),
- ein sich zum Röntgen-C-Bogen (4) hin erstreckender Ausleger (64) ist mit seinem Fixierende (67) mit der Tragvorrichtung verbunden und trägt mit seinem Freiende (66) den Stoßwellenkopf (6),
- der Ausleger (64) ist mit Hilfe der Tragvorrichtung derart beweglich geführt, **dadurch gekennzeichnet, dass** der Ausleger (64) derart beweglich geführt ist dass der Stoßwellenkopf (6) in der Orbitalebene (40) innerhalb eines Winkelbereiches von mindestens 180° ober- und unterhalb eines Patiententisches (10) beliebig positionierbar und auf das Isozentrum (32) ausrichtbar ist,
- der Ausleger (64) in einer zur Orbitalebene (40) des Röntgen-C-Bogens (4) parallelen Ebene zwangsgeführt ist,
- die Tragvorrichtung ein zum Röntgen-C-Bogen (4) axial versetzt und koaxial angeordneter C-Bogen (8) ist, an dem der Ausleger (64) mit seinem Fixierende (67) orbital verfahrbar gelagert ist, und
- der C-Bogen (8) orbital verfahrbar gelagert ist.

2. Anlage (2) nach Anspruch 1, bei der der Stoßwellenkopf (6) von einem für Röntgenstrahlen durchlässigen, sich längs seiner Stoßwellenachse (68) erstreckenden Zentralbereich (96) durchsetzt ist.

3. Anlage (2) nach einem der vorhergehenden Ansprüche, bei der der Röntgen-C-Bogen (4) angular verschwenkbar ist.

4. Anlage (2) nach einem der vorhergehenden Ansprüche, bei der die Tragvorrichtung zusammen mit dem Stoßwellenkopf (6) aus einer Behandlungsstellung in eine vom Patiententisch (10) bzw. einem darauf gelagerten Patienten (76) entfernte Parkposition verfahrbar ist.

5. Anlage (2) nach einem der vorhergehenden Ansprüche, mit einem den Röntgen-C-Bogen (4) durchsetzenden Patiententisch (10), der außerhalb des Verfahrbereichs von Röntgen-C-Bogen (4) und Tragvorrichtung gelagert ist.

## Claims

1. Installation (2) for image-supported shockwave treatment, with the following embodiment:
- it comprises an X-ray C-arm (4), which can be displaced in an orbit about an isocentre (32) and has an X-ray system (34, 36), a shockwave head (6) and a support device for the shockwave head (6) arranged laterally from, and stationary with respect to, the X-ray C-arm (4),
- a boom (64) that extends towards the X-ray C-arm (4) is connected to the support device at its fixed end (67) and carries the shockwave head (6) at its free end (66),
- the boom (64) is moveably guided with the aid of the support device, **characterized in that** the boom (64) is moveably guided such that the shockwave head (6) can be positioned anywhere in the orbital plane (40) within an angular range of at least 180° above and below a patient table (10) and aligned with respect to the isocentre (32),
- the boom (64) is forcibly guided in a plane parallel to the orbital plane (40) of the X-ray C-arm (4),
- the support device is a coaxially arranged C-arm (8), which is axially offset with respect to the X-ray C-arm (4) and on which the boom (64) is mounted with its fixed end (67) such that it can be displaced in its orbit, and
- the C-arm (8) is mounted such that it can be displaced in its orbit.

2. Installation (2) according to Claim 1, in which the shockwave head (6) is penetrated by a central region (96) that is permeable to X-ray radiation and extends along its shockwave axis (68).

3. Installation (2) according to one of the preceding claims, in which the X-ray C-arm (4) can be pivoted in an angular fashion.

4. Installation (2) according to one of the preceding claims, in which the support device together with the shockwave head (6) can be displaced from a treatment position into a park position which is at a distance from the patient table (10) or a patient (76) positioned thereon.

5. Installation (2) according to one of the preceding claims, with a patient table (10) penetrating the X-ray C-arm (4), which patient table is mounted outside of the displacement range of X-ray C-arm (4) and support device.

## Revendications

1. Installation ( 2 ) de traitement par ondes de choc assisté par images ayant la constitution suivante :
- elle comprend un arceau ( 4 ) en C à rayons X déplaçable orbitalement autour d'un isocentre ( 32 ) et ayant un système ( 34, 36 ) à rayons X, une tête ( 6 ) d'ondes de choc et un dispositif de support de la tête ( 6 ) d'ondes de choc monté latéralement et fixe par rapport à l'arceau (4) en C à rayons X,
- un bras ( 64 ) s'étendant vers l'arceau ( 4 ) en C à rayons X est relié par son extrémité ( 67 ) de fixation au dispositif de support et porte par son extrémité ( 66 ) libre la tête ( 6 ) d'ondes de choc,
- le bras ( 64 ) est guidé de manière mobile à l'aide du dispositif de support, **caractérisé en ce que** le bras ( 64 ) est guidé de manière mobile de façon à ce que la tête ( 6 ) d'ondes de choc puisse être mise en position à volonté dans le plan ( 40 ) orbital dans une plage angulaire d'au moins 180° au-dessus et en dessous d'une couchette ( 60 ) de patient dirigée sur l'isocentre ( 32 ),
- le bras ( 64 ) est obligé de se déplacer dans une direction parallèle au plan ( 40 ) orbital de l'arceau ( 4 ) en C à rayons X,
- le dispositif de support est un arceau ( 8 ) en C décalé axialement par rapport à l'arceau ( 4 ) en C à rayons X et disposé coaxialement, arceau ( 8 ) sur lequel le bras ( 64 ) est monté mobile orbitalement par son extrémité ( 67 ) de fixation, et
- l'arceau ( 8 ) en C est monté mobile orbitalement.

2. Installation ( 2 ) suivant la revendication 1, dans laquelle la tête ( 6 ) d'ondes de choc est traversée par une zone ( 96 ) centrale perméable aux rayons X et s'étendant le long de son axe ( 68 ) d'ondes de choc.

3. Installation ( 2 ) suivant l'une des revendications précédentes, dans laquelle l'arceau ( 4 ) en C à rayons X peut pivoter angulairement.

4. Installation ( 2 ) suivant l'une des revendications précédentes, dans laquelle le dispositif de support peut, ensemble avec la tête ( 6 ) d'ondes de choc, être déplacé d'une position de traitement à une position de repos éloignée de la couchette ( 10 ) pour le patient et d'un patient ( 76 ), qui y repose.

5. Installation ( 2 ) suivant l'une des revendications précédentes, comprenant une couchette ( 10 ) pour un patient, qui est traversée par l'anneau ( 4 ) en C à rayons X et qui est montée à l'extérieur de la zone de déplacement de l'arceau ( 4 ) en C à rayons X et du dispositif de support.
